(19) Europäisches Patentamt
European Patent Office
Office européen des brevets

(11) Numéro de publication: **0 427 606 B1**

(12) # FASCICULE DE BREVET EUROPEEN

(45) Date de publication de fascicule du brevet: **01.02.95**

(51) Int. Cl.[6]: **C07D 405/04**, C07D 491/04, A61K 31/44

(21) Numéro de dépôt: **90403124.2**

(22) Date de dépôt: **06.11.90**

Le dossier contient des informations techniques présentées postérieurement au dépôt de la demande et ne figurant pas dans le présent fascicule.

(54) **Dérivés d'amidino-4 chromanne et d'amidino-4 pyranno (3,2-c) pyridine, procédé d'obtention et compositions pharmaceutiques les contenant.**

(30) Priorité: **06.11.89 FR 8914518**
**02.02.90 FR 9001258**

(43) Date de publication de la demande:
**15.05.91 Bulletin 91/20**

(45) Mention de la délivrance du brevet:
**01.02.95 Bulletin 95/05**

(84) Etats contractants désignés:
**AT BE CH DE DK ES FR GB GR IT LI LU NL SE**

(56) Documents cités:
**EP-A- 0 205 292**
**EP-A- 0 296 975**
**EP-A- 0 312 432**
**EP-A- 0 344 747**

(73) Titulaire: **SANOFI**
**32-34, rue Marbeuf**
**F-75008 Paris (FR)**

(72) Inventeur: **Garcia, Georges**
**27, Rue des Aires**
**F-34980 Saint-Gely-Du-Fesc (FR)**
Inventeur: **Gautier, Patrick**
**Lieu dit Manavieille**
**F-34660 Cournonterral (FR)**
Inventeur: **Nisato, Dino**
**2, Rue de Terre Rouge**
**F-34680 Saint Georges d'Orques (FR)**
Inventeur: **Roux, Richard**
**9 Lotissement de l'Arnède**
**F-34570 Vailhauques (FR)**

(74) Mandataire: **Gillard, Marie-Louise et al**
**Cabinet Beau de Loménie**
**158, rue de l'Université**
**F-75340 Paris Cédex 07 (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

## Description

La présente invention a pour objet des dérivés d'amidino-4 chromanne et d'amidino-4 pyranno [3,2-c] pyridine, un procédé pour leur obtention et des compositions pharmaceutiques les contenant. Lesdits dérivés ont des activités antihypertensive et antiarythmique.

Le brevet belge 829 611 mentionne toute une série de dérivés du chromannol-3 à activité antihypertensive ; ces dérivés sont caractérisés par la présence en position 4 d'un groupe $NR_1R_2$ dans lequel $R_1$ est l'hydrogène ou un groupe hydrocarboné éventuellement substitué, $R_2$ est l'hydrogène ou un alkyle, $NR_1R_2$ pouvant être un groupe hétérocyclique comportant de 3 à 8 atomes, non substitué ou substitué par un ou deux groupes méthyle et par la présence éventuelle d'un grand nombre de substituants possibles en position 6 ou en position 7.

La demande de brevet européen n° 273 262 décrit des dérivés du chromanne de formule :

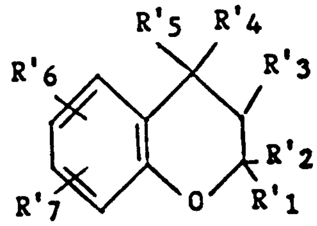

dans laquelle notamment :

- $R'_5$ désigne un groupe hétérocyclique tel que pyridone-2 yl-1, pyridazinone-2 yl-1, pyrimidone-2 yl-1, pyrimidone-6 yl-1, pyrazinone-2 yl-1, thiopyridone-2 yl-1 ; ce groupe pouvant être partiellement hydraté, non substitué ou substitué ;
- $R'_3$ représente OH ou $OCOCH_3$ ;
- $R'_4$ représente l'hydrogène ou bien $R'_3$ et $R'_4$ ensemble forment une liaison.

Les demandes de brevet européens 296 975 et 312 432 décrivent des composés semblables.

Enfin, la demande de brevet européen 205 292 décrit des dérivés de pyranno[3,2-c]pyridine de formule :

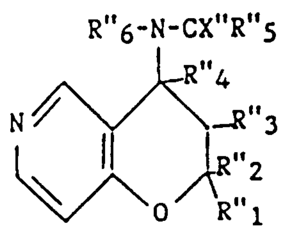

dans laquelle :
X″ représente O ou S et $R''_5$ et $R''_6$ peuvent former un hétérocycle.

On a maintenant trouvé des nouveaux composés, dérivés du chromanne ou du pyranno [3,2-c] pyridine qui présentent une activité comme antihypertenseurs et antiarythmiques.

Ainsi, la présente invention concerne selon un de ses aspects des composés de formule :

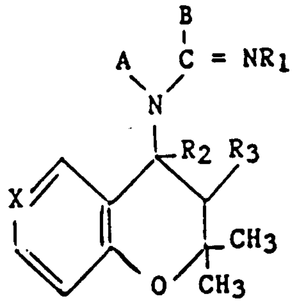

(I)

dans laquelle :

- A et B sont unis entre N et C = $NR_1$ et représentent
  . soit un groupe -CH = CH-CH = N-
  . soit un groupe -CH = $CR_4$-$CR_5$ = CH-, l'un des substituants $R_4$ ou $R_5$ représentant l'hydrogène, l'autre étant choisi parmi l'hydrogène, un halogène, un nitro ou un alkyle en $C_1$-$C_4$ ;
- X représente un atome d'azote, un groupement N-oxyde ou le groupe C-Z ;
- Z représente l'hydrogène, un halogène, un alkyle en $C_1$-$C_4$, un groupe cyano, un groupe nitro, un groupe acétyle ou un groupe trifluoroacétyle, un groupe phosphono, un groupe dialcoxyphosphoryle dans lequel l'alkyle est en $C_1$-$C_3$, ou un groupe amino.
- $R_1$ représente l'hydrogène, un groupe cyano, un groupe nitro, un alkyle en $C_1$-$C_4$, un hydroxyle, un alcoxy en $C_1$-$C_4$, un trifluoroacétyle, un groupe méthanesulfonyle, un groupe benzènesulfonyle non substitué ou substitué sur le phényle par un méthyle, un halogène ou un trifluorométhyle ;
- $R_2$ représente un atome d'hydrogène ;
- $R_3$ représente un groupe hydroxyle ou un groupe acétyloxy ou $R_2$ et $R_3$ forment ensemble une liaison.

Dans la présente description et dans les revendications qui vont suivre, par halogène, on entend un atome de chlore, de brome ou de fluor.

Les composés de formule (I) dans laquelle $R_2$ = H et $R_3$ = OH ou $OCOCH_3$ ont la configuration trans. Ils peuvent présenter deux carbones asymétriques. La présente invention comprend chacun des isomères optiques des composés de formule (I) ainsi que le racémique.

La présente invention a également pour objet un procédé pour la préparation des composés (I).

Ledit procédé est caractérisé en ce que :

a) on traite un époxyde II de formule :

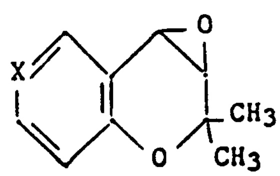

(II)

dans laquelle :

X a les significations données ci-dessus pour I avec un hétérocycle III répondant à l'une des formes tautomères suivantes :

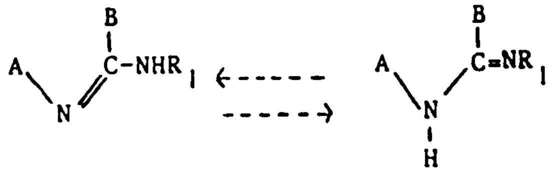

(III)

dans lesquelles :

A, B et $R_1$ ont les significations indiquées précédemment pour (I) ;

b) éventuellement, on fait réagir sur le composé obtenu de formule (I) dans laquelle $R_2$ = H et $R_3$ = OH l'anhydride acétique, pour préparer le composé de formule (I) dans laquelle $R_2$ = H et $R_3$ = $OCOCH_3$ ;

c) éventuellement, on traite le composé obtenu à l'étape a) de formule (I) dans laquelle $R_2$ = H et $R_3$ = OH ou le composé obtenu à l'étape b) de formule (I) dans laquelle $R_2$ = H et $R_3$ = $OCOCH_3$, pour préparer un composé de formule (I) dans laquelle $R_2$ et $R_3$ forment ensemble une liaison.

A l'étape a), la réaction d'ouverture de l'époxyde (II) est conduite à une température comprise entre 10°C et 100°C dans un solvant organique inerte comme le dioxanne, le tétrahydrofuranne, le méthyl-tert-butyléther, le diméthylsulfoxyde ou le diméthylformamide en présence d'un agent de condensation basique tel que l'hydrure de sodium, la triéthylamine, un hydroxyde d'ammonium quaternaire comme l'hydroxyde de benzyltriméthylammonium. Dans ces conditions opératoires, l'ouverture de l'époxyde (II) conduit à des composés de formule (I) dans lesquels $R_2$ = H et $R_3$ = OH ayant la configuration trans.

A l'étape b), l'acétylation est effectuée à une température comprise entre 20 et 100°C pendant une période allant de 1 à 48 h dans un solvant basique tel que la pyridine ou la triéthylamine.

A l'étape c) la déshydratation du composé préparé à l'étape a) est obtenue par action d'un hydrure alcalin, par exemple l'hydrure de sodium ou l'hydrure de lithium, dans un solvant inerte à une température comprise entre 20°C et 100°C ; la désacétylation du composé préparé à l'étape b) est obtenue en présence de diazabicycloundécène en chauffant entre 50 et 110°C dans un solvant inerte tel que le toluène ou le benzène.

Les époxydes de formule (II) sont connus ou préparés selon des méthodes connues.

L'époxyde (II) dans lequel X représente un atome d'azote est décrit dans la demande de brevet européen 205 292. L'époxyde (II) dans lequel X représente un groupement N-oxyde est décrit dans la demande WO 89/10925. Les époxydes (II) dans lesquels X représente le groupement C-Z sont décrits dans différentes publications.

Ainsi, l'époxyde (II) dans lequel Z représente le groupe cyano est décrit dans le brevet belge 852 955 ; l'époxyde (II) dans lequel Z représente le groupe nitro ou un groupe acétyle est décrit dans J. Med. Chem., 1983, 23, 1 582-1 589 ; les époxydes (II) dans lesquels Z représente un halogène sont préparés selon Tetrahedron, 1981, 37 (15), 2 613-2 616. Les époxydes (II) dans lesquels Z représente le trifluoroacétyle ou un groupe phosphono ou dialcoxyphosphoryle dans lequel l'alkyle est en $C_1$-$C_3$ sont décrits dans la demande de brevet européen 296 975. L'époxyde (II) dans lequel Z représente l'hydrogène est décrit dans J. Chem. Soc., 1962, 76-79. L'époxyde (II) dans lequel Z est un méthyle est décrit dans Aust. J. Chem. 1979, 32 (3), 619-636 ; lorsque Z représente un alkyle en $C_2$-$C_4$, l'époxyde (II) est obtenu de façon analogue. Enfin, l'époxyde (II) dans lequel Z représente un groupe amino est décrit dans la demande de brevet européen 0 273 262. Il peut aussi être obtenu par réduction de l'époxyde (II) dans lequel Z représente le groupe nitro.

Les composés hétérocycliques (III) sont connus ou préparés par des méthodes connues. En particulier, pour la préparation des cyanaminopyridines ou pyrimidines, on peut utiliser la méthode décrite dans Ann. Pharm. Fr., 1968, 26 (6), 469-472. Les sulfonaminopyridines sont décrites dans Doklady Akad. Nauk. S.S.S.R., 1957, 113, 1 080-3. Ils peuvent être préparés par action du dérivé sulfonyle approprié sur l'aminopyridine. Par ailleurs, on a appliqué des méthodes classiques de la chimie organique pour la préparation de certains composés (III). Ainsi, les composés (III) dans lesquels $R_1$ est un groupe nitro servent à préparer les composés (III) dans lesquels $R_1$ est un alkyle en $C_1$-$C_4$ par action d'une alkylamine en $C_1$-$C_4$ en milieu basique. De la même façon, ils peuvent être utilisés pour préparer les composés (III) dans lesquels $R_1$ est hydroxyle par action de l'hydroxylamine. Les pyridylnitramines ($R_1$ = $NO_2$) sont préparées selon J. Am. Chem. Soc., 1955, 77, 3 154- 3 155.

Les composés selon l'invention augmentent la polarisation des fibres musculaires lisses et ont un effet vasodilatateur sur la veine porte, selon le test décrit dans la demande de brevet européen 296 975. Leur effet antihypertenseur a été observé chez l'animal.

Par ailleurs, on a observé que les composés selon l'invention accélèrent la repolarisation des cellules myocardiques ; parallèlement, leur effet antiarythmique a été observé sur un modèle animal.

Aucun signe de toxicité n'est observé avec ces composés aux doses pharmacologiquement actives.

Ainsi, les composés selon l'invention peuvent être utilisés dans le traitement de l'hypertension, des troubles pathologiques associés aux contractions des fibres musculaires lisses des appareils gastrointestinal, respiratoire, utérin et urinaire, par exemple : ulcère, asthme, contraction utérine prématurée, incontinence, et dans le traitement d'autres troubles pathologiques cardiovasculaires tels que : angor, insuffisance cardiaque, maladies vasculaires cérébrales et périphériques. De plus, les composés selon l'invention peuvent être utilisés dans le traitement de l'arythmie cardiaque ainsi que dans le traitement du glaucome.

Par ailleurs, les composés selon l'invention peuvent être utilisés dans le traitement de la dépression ou d'autres maladies du système nerveux central, l'épilepsie par exemple.

Enfin, les composés de la présente invention peuvent être utilisés pour le traitement topique de l'alopécie.

La présente invention a donc également pour objet des compositions pharmaceutiques contenant une dose efficace d'un composé selon l'invention et des excipients convenables. Lesdits excipients sont choisis selon la forme pharmaceutique et le mode d'administration souhaité.

Dans les compositions pharmaceutiques de la présente invention pour l'administration orale, sublinguale, sous-cutanée, intramusculaire, intraveineuse, topique, transdermique ou rectale, les principes actifs de formule (I) ci-dessus peuvent être administrés sous formes unitaires d'administration, en mélange avec des supports pharmaceutiques classiques, aux animaux et aux êtres humains pour la prophylaxie ou le traitement des troubles ou des maladies ci-dessus. Les formes unitaires d'administration appropriées comprennent les formes par voie orale telles que les comprimés, les gélules, les poudres, les granules et les solutions ou suspensions orales, les formes d'administration sublinguale et buccale, les formes d'administration sous-cutanée, intramusculaire ou intraveineuse et les formes d'administration rectale. Pour l'application topique, on peut utiliser les composés selon l'invention dans des crèmes, pommades ou lotions.

Afin d'obtenir l'effet prophylactique ou thérapeutique désiré, la dose de principe actif peut varier entre 0,01 et 1 mg par kg de poids du corps et par jour.

Chaque dose unitaire peut contenir de 0,01 à 2 mg, de préférence de 0,02 à 1 mg, d'ingrédients actifs en combinaison avec un support pharmaceutique. Cette dose unitaire peut être administrére 1 à 5 fois par jour de façon à administrer un dosage journalier de 0,01 à 10 mg, de préférence de 0,02 à 5 mg.

Lorsque l'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose d'un dérivé cellulosique, ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir ou pour l'administration sous forme de gouttes peut contenir l'ingrédient actif conjointement avec un édulcorant, acalorique de préférence, du méthylparaben et du propylparaben comme antiseptiques, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Les compositions de la présente invention peuvent contenir, à côté des produits de formule I ci-dessus ou d'un de leurs sels pharmaceutiquement acceptables, d'autres principes actifs tels que, par exemple, des tranquillisants, des bêta-bloquants ou d'autres médicaments qui peuvent être utiles dans le traitement des troubles ou maladies indiquées ci-dessus.

Les exemples suivants illustrent l'invention sans toutefois la limiter. Dans les exemples, ainsi que dans la partie descriptive et dans les revendications, certains produits sont désignés comme dérivés du chromanne. Il est entendu que ces produits de la présente invention sont des dérivés du diméthyl-2,2 dihydro-3,4 2H- chromène et que le terme "chromanne" désigne le "dihydro-3,4 2H- chromène".

Les points de fusion (Fc) sont donnés en degrés Celsius.

Exemple 1

Trans(cyanimino-2 dihydro-1,2 pyridyl-1)-4 cyano-6 diméthyl-2,2 hydroxy-3 chromanne : SR 47023. (A + B = -CH = CH-CH = CH- ; X = C-CN ; $R_1$ = CN ; $R_2$ = H ; $R_3$ = OH).

La cyanamino-2 pyridine est préparée selon la méthode décrite dans Ann. Pharm. Fr., 1968, 26 (6), 469-472.

On chauffe à reflux pendant 24 h une solution contenant 1 g de cyano-6 diméthyl-2,2 époxy-4,4 chromanne, 0,8 g de cyanamino-2 pyridine dans 50 ml de diméthoxyéthane et 0,2 ml d'hydroxyde de benzyltriméthylammonium. On concentre le milieu réactionnel puis on reprend par 50 ml d'eau et on extrait 2 fois à l'acétate d'éthyle. On sèche sur sulfate de sodium puis concentre. Le résidu est repris dans l'éther éthylique, filtré puis lavé par du dichlorométhane. On obtient 110 mg du produit attendu.
Fc = 284 °C.

Exemple 2

Trans(cyanimino-2 dihydro-1,2 pyridyl-1)-4 diméthyl-2,2 hydroxy-3 nitro-6 chromanne : SR 47025. (A + B = -CH=CH-CH=CH- ; X = C-$NO_2$ ; $R_1$ = CN ; $R_2$ = H ; $R_3$ = OH).

On chauffe à reflux pendant 96 h une solution contenant 2,7 g de cyanamino-2 pyridine, 3 g de diméthyl-2,2 époxy-3,4 nitro-6 chromanne dans 50 ml de tétrahydrofuranne et 0,2 ml de triéthylamine. On laisse refroidir puis on filtre. Le précipité est lavé à l'éther éthylique puis à l'eau et à l'acétone et séché. On obtient 250 mg du produit attendu.
Fc = 253 °C.

Exemple 3

(Cyanimino-2 dihydro-1,2 pyridyl-1)-4 diméthyl-2,2 nitro-6 chromène : SR 47063. (A + B = -CH=CH-CH=CH- ; X = C-$NO_2$ ; $R_1$ = CN ; $R_2$ + $R_3$ = double liaison).

Ce composé est préparé à partir du chromannol obtenu à l'exemple 2.

A 400 mg de SR 47025 placés dans 50 ml de tétrahydrofuranne, on ajoute par petites fractions 30 mg d'hydrure de sodium. Après 96 h à température ambiante, on concentre le milieu réactionnel à sec puis on reprend par 50 ml d'eau; On extrait 2 fois par du chlorure de méthylène puis on sèche et concentre. On obtient 250 mg du produit qui sont purifiés par chromatographie sur colonne de silice en éluant par un mélange chlorure de méthylène-méthanol (99,5/0,5 - v/v).

On recueille 80 mg du produit attendu.
Fc = 257 °C.

Exemple 4

Trans acétyloxy-3 (cyanimino-2 dihydro-1,2 pyridyl-1)-4 diméthyl-2,2 nitro-6 chromanne : SR 47601. (A + B = -CH=CH-CH=CH- ; X = C-$NO_2$ ; $R_1$ = CN ; $R_2$ = H ; $R_3$ = $OCOCH_3$).

On délaye 5 g de SR 47025, obtenu à l'exemple 2, dans 25 ml de pyridine, on ajoute 3,8 ml d'anhydride acétique et on laisse sous agitation à température ambiante pendant 4 h. Le mélange est repris par de l'acétate d'éthyle puis lavé à l'eau acide et à l'eau bicarbonatée. Le produit qui précipite est filtré, lavé à l'éther éthylique puis séché à 100 °C sous vide. On obtient 5,2 g du produit attendu.
Fc = 232 °C.

Exemple 5

(Cyanimino-2 dihydro-1,2 pyridyl-1)-4 diméthyl-2,2 nitro-6 chromène : SR 47063. (A + B = -CH=CH-CH=CH- ; X = C-$NO_2$ ; $R_1$ = CN ; $R_2$ + $R_3$ = double liaison).

On chauffe à reflux pendant 3 h un mélange contenant 5 g de SR 47061, obtenu à l'exemple 4, dans 100 ml de toluène, en présence de 2,8 ml de diazabicycloundécène. Le milieu réactionnel est filtré à température ambiante puis lavé au toluène et à l'éther éthylique. Le produit obtenu (4,1 g) est recristallisé dans 200 ml d'alcool et on obtient 3 g du produit attendu.
Fc = 257 °C.

Exemple 6

Trans acétyl-6 (cyanimino-2 dihydro-1,2 pyrimidyl-1)-4 diméthyl-2,2 hydroxy-3 chromanne : SR 47141 (A + B = -CH=CH-CH=N- ; X=-C-CO-$CH_3$ : $R_1$ = CN ; $R_2$ = H ; $R_3$ = OH).

La cyanamino-2 pyrimidine est préparée selon le brevet britannique 860 423.

Le SR 47141 est obtenu selon la méthode décrite à l'exemple 1.
Fc = 254 °C.

Exemple 7

Trans (cyanimino-2 dihydro-1,2 pyrimidyl-1)-4 diméthyl-2,2 hydroxy-3 nitro-6 chromanne : SR 47162. (A + B = -CH=CH-CH=N- ; X = C-$NO_2$ ; $R_1$ = CN ; $R_2$ = H ; $R_3$ = OH).

Ce composé est préparé en suivant le mode opératoire décrit à l'exemple 6.
Fc = 316 °C.

Exemple 8

Trans (cyanimino-2 dihydro-1,2 pyridyl-1)-4 diméthyl-2,2 hydroxy-3 trifluoroacétyl-6 chromanne : SR 47140. (A + B = -CH=CH-CH= CH- ; X=-C-CO-$CF_3$ ; $R_1$ = CN ; $R_2$ = H ; $R_3$ = OH).

Ce composé est préparé selon la méthode décrite à l'exemple 1.
Fc = 218 °C.

Exemple 9

Trans cyano-6 (dihydro-1,2 nitrimino-2 pyridyl-1)-4 diméthyl-2,2 hydroxy-3 chromanne : SR 47159. (A + B = -CH=CH-CH=CH- ; X = C-CN ; $R_1$ = $NO_2$ ; $R_2$ = H ; $R_3$ = OH).

La pyridylnitramine est préparée selon la méthode décrite dans J. Am. Chem. Soc., 1955, 77, 3154.

Le composé de l'exemple 9 est obtenu en suivant le procédé de l'exemple 2.
Fc = 279 °C.

Exemple 10

Trans-(cyanimino-2 dihydro-1,2 pyridyl-1)-4 dihydro-3,4 diméthyl-2,2 hydroxy-3 pyranno [3,2-c] pyridine : SR 47142. (A + B = -CH=CH-CH=CH- ; X = N ; $R_1$ = CN ; $R_2$ = H ; $R_3$ = OH).

Ce composé est obtenu à partir de l'époxyde (II) dans lequel X = N et de la cyanamino-2 pyridine.
Fc = 171 °C.

Exemple 11

(Cyanimino-2 dihydro-1,2 pyridyl-1)-4 diméthyl-2,2 pyranno [3,2-c] pyridine : SR 47320 (A + B = -CH=CH-CH=CH- ; X = N ; $R_1$ = CN ; $R_2$ + $R_3$ = double liaison).

On maintient au reflux pendant 5 h un mélange de SR 47142, préparé précédemment, 0,6 g de tétrahydrofuranne et 50 mg d'hydrure de sodium. On concentre, reprend par de l'eau, lave, sèche et concentre pour obtenir 0,45 g d'un solide jaune. On effectue ensuite une chromatographie sur silice en éluant par un mélange chlorure de méthylène-méthanol (99/1 - v/v). On recueille 90 mg du produit attendu.
Fc = 256 °C.

Exemple 12

(Cyanimino-2 dihydro-1,2 pyridyl-1)-4 cyano-6 diméthyl-2,2 chromène : SR 47164 (A + B = -CH=CH-CH=CH- ; X = C-CN ; $R_1$ = CN ; $R_2$ + $R_3$ = double liaison).

A) Trans (cyanimino-2 dihydro-1,2 pyridyl-1)-4 cyano-6 diméthyl-2,2 hydroxy-3 chromanne : préparé à l'exemple 1.

B) SR 47164

A 1 g de chromannol préparé précédemment placé dans 50 ml de tétrahydrofuranne anhydre, on ajoute par petites fractions 80 mg d'hydrure de sodium. Après 2 h à reflux, on concentre le milieu réactionnel à sec puis on reprend par 100 ml d'eau. On extrait 2 fois par du chlorure de méthylène puis on sèche et concentre. On obtient 600 mg de produit qui sont purifiés par chromatographie sur colonne de silice en éluant par un mélange chlorure de méthylèneméthanol (99,5/0,5 - v/v).

On recueille 400 mg du produit attendu.
Fc = 292 °C.

Exemple 13

Trans diméthyl-2,2-hydroxy-3 (méthanesulfonimino-2 dihydro-1,2 pyridyl-1)-4 nitro-6 chromanne : SR 47195.
(A + B = -CH=CH-CH=CH- ; X = $C-NO_2$ ; $R_1$ = $CH_3-SO_2-$ ; $R_2$ = H ; $R_3$ = OH).

On refroidit à 0°C une solution de 4,7 g d'amino--2 pyridine dans 6,3 ml de pyridine et on ajoute goutte à goutte 5,75 g de chlorure de mésyle. On laisse sous agitation 1 h à 0°C puis 24 h à température ambiante. Le mélange réactionnel solidifié est versé dans 20 ml d'eau et on laisse sous agitation pour déliter le précipité. Après essorage, le produit solide recueilli est recristallisé dans l'eau. On obtient 6,7 g de méthanesulfonimino-2 pyridine.
Fc = 204°C.

On procède ensuite selon le mode opératoire habituel par action sur le diméthyl-2,2 époxy-3,4 nitro-6 chromanne pour obtenir le produit attendu.
Fc = 265-267°C.

Exemple 14

Trans diméthyl-2,2 hydroxy-3 (dihydro-1,2 nitrimino-2 pyridyl-1)-4 nitro-6 chromanne : SR 47174. (A + B = -CH=CH-CH=CH- ; X = $C-NO_2$ ; $R_1$ = $NO_2$ ; $R_2$ = H ; $R_3$ = OH).

Ce composé est préparé selon l'exemple 9.
Fc=269°C.

Exemple 15

Trans (dihydro-1,2 méthylimino-2 pyridyl-1)-4 diméthyl-2,2 hydroxy-3 nitro-6 chromanne : SR 47220.
(A + B = -CH=CH-CH=CH- ; X = $C-NO_2$ ; $R_1$ = $CH_3$ ; $R_2$ = H ; $R_3$ = OH).

Dans une solution d'éthanol à 30 %, on mélange 500 mg de SR 47174, préparé à l'exemple précédent, et 50 ml de méthylamine et on laisse à température ambiante pendant 3 jours. On filtre l'insoluble, chasse les solvants, reprend dans de l'éther éthylique, dissout le précipité et filtre l'insoluble. On lave la phase éthérée à l'eau et sèche sur sulfate de sodium. On dissout à chaud dans l'éther isopropylique, puis le produit attendu cristallise.
m = 60 mg.
Fc = 155°C.

Exemple 16

Trans [dihydro-1,2 (trifluorométhyl-2 phényl)sulfonimino-2 pyridyl-1]-4 diméthyl-2,2 hydroxy-3 nitro-6 chromanne : SR 47281. (A + B = -CH=CH-CH=CH- ; X = $C-NO_2$,

$R_1$ = 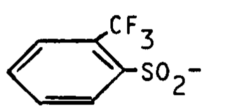 ;

$R_2$ = H ; $R_3$ = OH).

On refroidit, à 0°C, 0,94 g d'amino-2 pyridine dans 5 ml de pyridine, on ajoute 2,5 g de chlorure de trifluorométhyl-2 phénylsulfonyle et on laisse sous agitation pendant une nuit à température ambiante. Le mélange réactionnel est versé dans l'eau et on extrait par du chlorure de méthylène. La phase organique est lavée à l'eau puis séchée sur sulfate de sodium et concentrée sous vide. Le résidu solide est repris dans l'acétate d'éthyle et essoré. On obtient 2 g de (trifluorométhyl-2 phényl) sulfonimino-2 pyridine, sous forme d'un solide blanc.
Fc = 218-220°C.

On prépare ensuite le SR 47281 par action sur le diméthyl-2,2 époxy-3,4 nitro-6 chromanne, selon la méthode décrite dans les exemples précédents.
Fc = 305°C.

Exemples 17 à 35

D'autres composés selon l'invention ont été préparés en utilisant le mode opératoire décrit dans les exemples précédents. Ces composés sont rassemblés dans les tableaux 1 et 2 ci-dessous.

TABLEAU 1

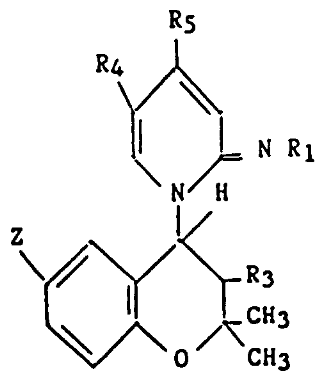

(I)

| n° SR Exemple n° | Z | $R_1$ | $R_4$, $R_5$ | $R_3$ | F°C |
|---|---|---|---|---|---|
| 47163<br>17 | $NO_2$ | H | H,H | OH | 184 |
| 47219<br>18 | CN | $CF_3$ | H,H | OH | 233 |
| 47282<br>19 | CN | CN | Cl,H | OH | 314 |
| 47283<br>20 | CN | CN | H,$CH_3$ | OH | 268 |
| 47321<br>21 | CN | $OCH_3$ | $NO_2$,H | OH | 164 |
| 47416<br>22 | $NO_2$ | CN | H,$CH_3$ | OH | 292 |
| 47417<br>23 | CN | CN | $CH_3$,H | OH | 309 |

TABLEAU 1 (suite)

| n° SR Exemple n° | Z | $R_1$ | $R_4$, $R_5$ | $R_3$ | F°C |
|---|---|---|---|---|---|
| 47433 24 | $NO_2$ | OH | H,H | OH | 219 |
| 47599 25 | $NO_2$ | $NO_2$ | H,H | $OCOCH_3$ | 237 |
| 47601 26 | $NO_2$ | CN | H,H | $OCOCH_3$ | 235 |
| 47602 27 | $COCF_3$ | CN | H,H | $OCOCH_3$ | 125 |
| 47603 28 | Br | CN | H,H | OH | 277 |
| 47604 29 | Br | CN | H,H | $OCOCH_3$ | 217 |
| 47605 30 | $CH_3$ | CN | H,H | $OCOCH_3$ | 218 |

TABLEAU 2

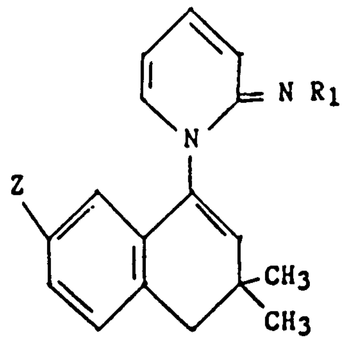

| n° SR<br>Exemple n° | Z | $R_1$ | F°C |
|---|---|---|---|
| 47319<br>31 | CN | CN | 252 |
| 47600<br>32 | $NO_2$ | $NO_2$ | 237 |
| 47617<br>33 | $COCF_3$ | CN | 190 |
| 47618<br>34 | Br | CN | 250 |
| 48259<br>35 | $NH_2$ | CN | 266 |

**Revendications**

**Revendications pour les Etats contractants suivants : AT, BE, CH, DE, DK, FR, GB, IT, LI, LU, NL, SE**

**1.** Composé de formule :

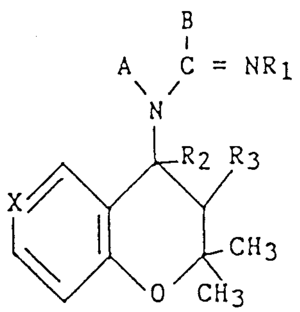

(I)

dans laquelle :

- A et B sont unis entre N et C = $NR_1$ et représentent
  . soit un groupe -CH=CH-CH=N-
  . soit un groupe $-CH=CR_4-CR_5=CH-$, l'un des substituants $R_4$ ou $R_5$ représentant l'hydrogène, l'autre étant choisi parmi l'hydrogène, un halogène, un nitro ou un alkyle en $C_1$ -$C_4$ ;
- X représente un atome d'azote, un groupement N-oxyde ou le groupe C-Z ;
- Z représente l'hydrogène, un halogène, un alkyle en $C_1$-$C_4$, un groupe cyano, un groupe nitro, un groupe acétyle ou un groupe trifluoroacétyle, un groupe phosphono ou dialcoxyphosphoryle, le groupe alkyle étant en $C_1$-$C_3$, ou un groupe amino ;
- $R_1$ représente l'hydrogène, un groupe cyano, un groupe nitro, un alkyle en $C_1$-$C_4$, un hydroxyle, un alcoxy en $C_1$-$C_4$, un trifluoroacétyle, un groupe méthanesulfonyle, un groupe benzènesulfonyle non substitué ou substitué sur le phényle par un méthyle, un halogène ou un trifluorométhyle ;
- $R_2$ représente un atome d'hydrogène ;
- $R_3$ représente un groupe hydroxyle ou un groupe acétyloxy ou $R_2$ et $R_3$ forment ensemble une liaison.

**2.** Composé selon la revendication 1, de formule (I), dans laquelle :

- $R_1$ représente CN,
- X représente le groupe C-$NO_2$ ou le groupe C-CN,
- A et B représentent un groupe $-CH=CR_4-CR_5=CH-$ et
- $R_2$ et $R_3$ forment ensemble une liaison.

**3.** Composé selon l'une des revendications 1 ou 2, de formule (I), caractérisé en ce qu'il s'agit du (cyanimino-2 dihydro-1,2 pyridyl-1)-4 diméthyl-2,2 nitro-6 chromène.

**4.** Composé selon l'une des revendications 1 ou 2, de formule (I), caractérisé en ce qu'il s'agit du (cyanimino-2 dihydro-1,2 pyridyl-1)-4 cyano-6 diméthyl-2,2 chromène.

**5.** Procédé pour la préparation d'un composé selon l'une quelconque des revendications 1 à 4, caractérisé en ce que :

a) l'on traite un époxyde II de formule :

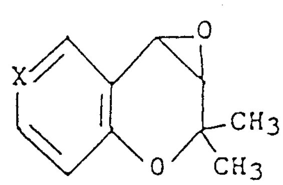

(II)

dans laquelle :

X a la signification donnée à la revendication 1 avec un hétérocycle III répondant à l'une des formes tautomères suivantes :

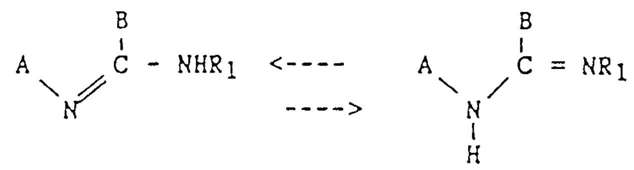

(III)

dans lesquelles :

A, B et $R_1$ ont les significations indiquées à la revendication 1,

b) éventuellement, on fait agir sur le composé obtenu de formule (I) dans laquelle $R_2$ = H et $R_3$ = OH, l'anhydride acétique pour préparer le composé de formule (I) dans laquelle $R_2$ = H et $R_3$ = $OCOCH_3$ ;

c) éventuellement, on traite le composé obtenu à l'étape a) de formule (I) dans laquelle $R_2$ = H et $R_3$ = OH, ou le composé obtenu à l'étape b) de formule (I) dans laquelle $R_2$ = H et $R_3$ = $OCOCH_3$, pour préparer un composé de formule (I) dans laquelle $R_2$ et $R_3$ forment ensemble une liaison.

**6.** Composition pharmaceutique caractérisée en ce qu'elle contient à titre de principe actif un composé de formule (I) selon l'une quelconque des revendications 1 à 4.

**7.** Composition pharmaceutique selon la revendication 6, contenant 0,01 à 2 mg de principe actif par unité de dosage.

**Revendications pour l'Etat contractant suivant : GR**

**1.** Composé de formule :

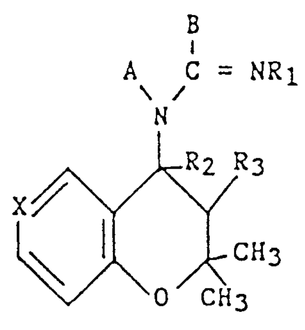

(I)

dans laquelle :

- A et B sont unis entre N et C = $NR_1$ et représentent
  . soit un groupe -CH=CH-CH=N-
  . soit un groupe $-CH=CR_4-CR_5=CH-$, l'un des substituants $R_4$ ou $R_5$ représentant l'hydrogène, l'autre étant choisi parmi l'hydrogène, un halogéne, un nitro ou un alkyle en $C_1$-$C_4$ ;
- X représente un atome d'azote, un groupement N-oxyde ou le groupe C-Z ;
- Z représente l'hydrogène, un halogène, un alkyle en $C_1$-$C_4$, un groupe cyano, un groupe nitro, un groupe acétyle ou un groupe trifluoroacétyle, un groupe phosphono ou dialcoxyphosphoryle, le groupe alkyle étant en $C_1$-$C_3$, ou un groupe amino ;
- $R_1$ représente l'hydrogène, un groupe cyano, un groupe nitro, un alkyle en $C_1$-$C_4$, un hydroxyle, un alcoxy en $C_1$-$C_4$, un trifluoroacétyle, un groupe méthanesulfonyle, un groupe benzènesulfonyle non substitué ou substitué sur le phényle par un méthyle, un halogène ou un trifluorométhyle ;
- $R_2$ représente un atome d'hydrogène ;
- $R_3$ représente un groupe hydroxyle ou un groupe acétyloxy ou $R_2$ et $R_3$ forment ensemble une liaison.

**2.** Composé selon la revendication 1, de formule (I), dans laquelle :
- $R_1$ représente CN,
- X représente le groupe C-$NO_2$ ou le groupe C-CN,
- A et B représentent un groupe -CH = $CR_4$-$CR_5$ = CH- et
- $R_2$ et $R_3$ forment ensemble une liaison.

**3.** Compose selon l'une des revendications 1 ou 2, de formule (I), caractérisé en ce qu'il s'agit du (cyanimino-2 dihydro-1,2 pyridyl-1)-4 diméthyl-2,2 nitro-6 chromène.

**4.** Composé selon l'une des revendications 1 ou 2, de formule (I), caractérisé en ce qu'il s'agit du (cyanimino-2 dihydro-1,2 pyridyl-1)-4 cyano-6 diméthyl-2,2 chromène.

**5.** Procédé pour la préparation d'un composé selon l'une quelconque des revendications 1 à 4, caractérisé en ce que :

a) l'on traite un époxyde II de formule :

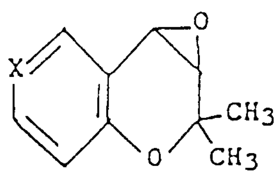

(II)

dans laquelle :

X a la signification donnée à la revendication 1 avec un hétérocycle III répondant à l'une des formes tautomères suivantes :

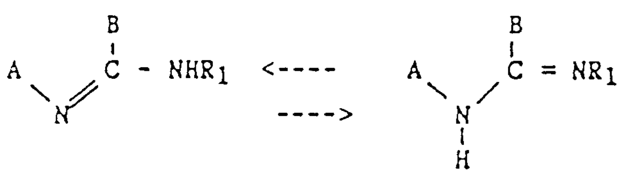

(III)

dans lesquelles :

A, B et $R_1$ ont les significations indiquées à la revendication 1,

b) éventuellement, on fait agir sur le composé obtenu de formule (I) dans laquelle $R_2$ = H et $R_3$ = OH, l'anhydride acétique pour préparer le composé de formule (I) dans laquelle $R_2$ = H et $R_3$ = $OCOCH_3$ ;

c) éventuellement, on traite le composé obtenu à l'étape a) de formule (I) dans laquelle $R_2$ = H et $R_3$ = OH, ou le composé obtenu a l'étape b) de formule (I) dans laquelle $R_2$ = H et $R_3$ = $OCOCH_3$, pour préparer un composé de formule (I) dans laquelle $R_2$ et $R_3$ forment ensemble une liaison.

**6.** Procédé pour la préparation de compositions pharmaceutiques, caractérisé en ce qu'il consiste à inclure une quantité efficace d'un composé de formule (I) selon l'une quelconque des revendications 1 à 4, dans un excipient convenable.

**7.** Procédé selon la revendication 6 pour la préparation decompositions pharmaceutiques contenant 0,01 à 2 mg de principe actif par unité de dosage.

**Revendications pour l'Etat contractant suivant : ES**

1. Procédé pour la préparation de composés de formule :

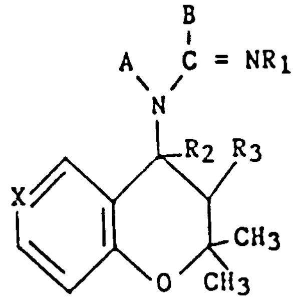

(I)

dans laquelle :

- A et B sont unis entre N et C = $NR_1$ et représentent
  . soit un groupe -CH=CH-CH=N-
  . soit un groupe $-CH=CR_4-CR_5=CH-$, l'un des substituants $R_4$ ou $R_5$ représentant l'hydrogène, l'autre étant choisi parmi l'hydrogène, un halogène, un nitro ou un alkyle en $C_1$-$C_4$ ;
- X représente un atome d'azote, un groupement N-oxide ou le groupe C-Z ;
- Z représente l'hydrogène, un halogène, un alkyle en $C_1$-$C_4$, un groupe cyano, un groupe nitro, un groupe acétyle ou un groupe trifluoroacétyle, un groupe phosphono ou dialcoxyphosphoryle, le groupe alkyle étant en $C_1$-$C_3$, ou un groupe amino ;
- $R_1$ représente l'hydrogène, un groupe cyano, un groupe nitro, un alkyle en $C_1$-$C_4$, un hydroxyle, un alcoxy en $C_1$-$C_4$, un trifluoroacétyle, un groupe méthanesulfonyle, un groupe benzènesulfonyle non substitué ou substitué sur le phényle par un méthyle, un halogène ou un trifluorométhyle ;
- $R_2$ représente un atome d'hydrogène ;
- $R_3$ représente un groupe hydroxyle ou un groupe acétyloxy ou $R_2$ et $R_3$ forment ensemble une liaison,

caractérisé en ce que :

a) l'on traite un époxyde II de formule :

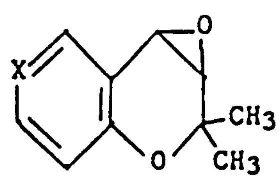

(II)

dans laquelle :

X a la signification donnée ci-dessus avec un hétérocycle III répondant à l'une des formes tautomères suivantes :

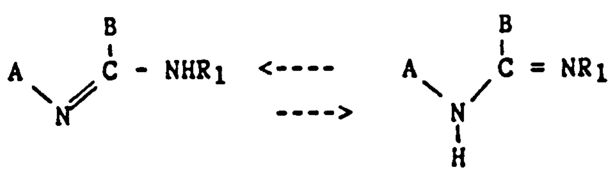

(III)

dans lesquelles :

A, B et $R_1$ ont les significations indiquées ci-dessus,

b) éventuellement, on fait agir sur le composé obtenu de formule (I) dans laquelle $R_2$ = H et $R_3$ = OH, l'anhydride acétique pour préparer le composé de formule (I) dans laquelle $R_2$ = H et $R_3$ =

$OCOCH_3$ ;
c) éventuellement, on traite le composé obtenu à l'étape a) de formule (I) dans laquelle $R_2$ = H et $R_3$ = OH, ou le composé obtenu à l'étape b) de formule (I) dans laquelle $R_2$ = H et $R_3$ = $OCOCH_3$, pour préparer un composé de formule (I) dans laquelle $R_2$ et $R_3$ forment ensemble une liaison.

**2.** Procédé selon la revendication 1, caractérisé en ce que l'on traite un époxyde de formule (II) dans laquelle X représente le groupe $C\text{-}NO_2$ ou le groupe C-CN avec un hétérocycle de formule (III) dans laquelle A et B représentent un groupe $-CH=CR_4\text{-}CR_5=CH-$, $R_1$ représente le groupe CN, pour préparer les composés de formule (I) dans laquelle :
- $R_1$ représente CN,
- X représente le groupe $C\text{-}NO_2$ ou le groupe C-CN,
- A et B représentent un groupe $-CH=CR_4\text{-}CR_5=CH-$ et
- $R_2$ et $R_3$ forment ensemble une liaison.

**3.** Procédé selon la revendication 1, caractérisé en ce que :
a) l'on traite un époxyde de formule (II) dans laquelle X représente le groupe $C\text{-}NO_2$ avec un hétérocycle de formule (III) dans laquelle A et B représentent le groupe -CH=CH-CH=CH- et $R_1$ représente le groupe CN ;
b) éventuellement, on fait réagir sur le composé ainsi obtenu de formule (I) dans laquelle $R_2$ = H et $R_3$ = OH, l'anhydride acétique pour préparer le composé de formule (I) dans laquelle $R_2$ = H et $R_3$ = $OCOCH_3$ ;
c) l'on traite le composé obtenu à l'étape a) avec un hydrure alcalin en solvant inerte à une température comprise entre 20 et 100 °C ou éventuellement le composé obtenu à l'étape b) par chauffage entre 50 et 110 °C dans un solvant inerte en présence de diazabicycloundécène pour obtenir le (cyanimino-2 dihydro-1,2 pyridyl-1)-4 diméthyl-2,2 nitro-6 chromène.

**4.** Procédé selon la revendication 1, caractérisé en ce que
a) l'on traite un époxyde de formule (II) dans laquelle X représente le groupe C-CN avec un hétérocycle de formule (III) dans laquelle A et B représentent le groupe -CH=CH-CH=CH- et $R_1$ représente le groupe CN,
b) éventuellement, on fait réagir sur le composé ainsi obtenu de formule (I) dans laquelle $R_2$ = H et $R_3$ = OH, l'anhydride acétique pour préparer le composé de formule (I) dans laquelle $R_2$ = H et $R_3$ = $OCOCH_3$ ;
c) l'on traite le composé obtenu à l'étape a) avec un hydrure alcalin en solvant inerte à une température comprise entre 20 et 100 °C ou éventuellement le composé obtenu à l'étape b) par chauffage entre 50 et 110 °C dans un solvant inerte en présence de diazabicycloundécène pour obtenir le (cyanimino-2 dihydro-1,2 pyridyl-1)-4 cyano-6 diméthyl-2,2 chromène.

**5.** Procédé pour la préparation de compositions pharmaceutiques caractérisé en ce qu'il consiste à inclure une quantité efficace d'un composé de formule (I), préparé selon l'une quelconque des revendications 1 à 4, dans un excipient convenable.

**6.** Procédé selon la revendication 5 pour la préparation de compositions pharmaceutiques contenant 0,01 à 2 mg de principe actif par unité de dosage.

## Claims

**Claims for the following Contracting States : AT, BE, CH, DE, DK, FR, GB, IT, LI, LU, NL, SE**

**1.** Compound of formula :

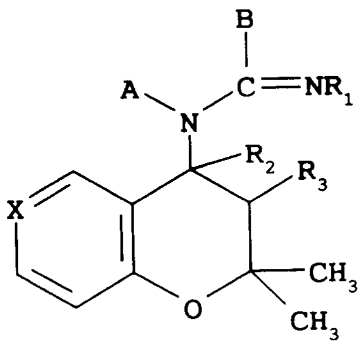

(I)

in which :

- A and B are linked together between N and $C=NR_1$ and represent
  - . either a -CH=CH-CH=N- group
  - . or a $-CH=CR_4-CR_5=CH-$ group, one of the substituents $R_4$ or $R_5$ denoting hydrogen, the other being selected from hydrogen, halogen, nitro or $C_1$-$C_4$ alkyl;
- X represents a nitrogen atom, a N-oxide group or the C-Z group;
- Z represents hydrogen, a halogen, a $C_1$-$C_4$ alkyl, a cyano group, a nitro group, an acetyl group or a trifluoroacetyl group, a phosphono or dialkoxyphosphoryl group, the alkyl group being a $C_1$-$C_3$ group or an amino group;
- $R_1$ represents hydrogen, a cyano group, a nitro group, a $C_1$-$C_4$ alkyl, an hydroxyl, a $C_1$-$C_4$ alkoxy, a trifluoroacetyl, a methanesulfonyl group, a benzensulfonyl group unsubstituted or substituted on the phenyl by a methyl, a halogen or a trifluoromethyl;
- $R_2$ represents hydrogen;
- $R_3$ represents an hydroxyl or acetyloxy group or $R_2$ and $R_3$ together form a bond.

**2.** Compound according to claim 1, of formula (I), in which :

- $R_1$ represents CN,
- X represents the C-$NO_2$ group or the C-CN group,
- A and B represents a $-CH=CR_4-CR_5=CH-$ group and
- $R_2$ and $R_3$ together form a bond.

**3.** Compound according to one of claims 1 or 2, of formula (I), characterized in that said compound is 4-(2-cyanimino 1,2-dihydro 1-pyridyl) 2,2-dimethyl 6-nitro chromene.

**4.** Compound according to one of claims 1 or 2, of formula (I), characterized in that said compound is 4-(2-cyanimino 1,2-dihydro 1-pyridyl) 6-cyano 2,2-dimethyl chromene.

**5.** Process for the preparation of a compound according to any one of claims 1 to 4, characterized in that :

a) an epoxide II of formula :

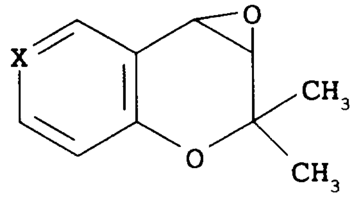

(II)

in which X has the meaning given in claim 1,is treated with a heterocycle III corresponding to one of

the following tautomeric forms :

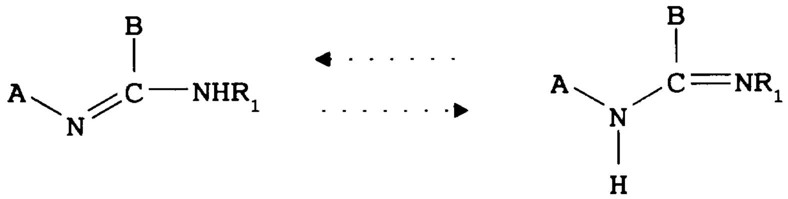

(III)

in which A, B and $R_1$ have the meanings indicated in claim 1,

b) acetic anhydride is optionally reacted with the compound obtained of formula (I) in which $R_2$ = H and $R_3$ = OH in order to prepare the compound of formula (I) in which $R_2$ = H and $R_3$ = $OCOCH_3$;

c) the compound obtained in step a) of formula (I) in which $R_2$ = H and $R_3$ = OH or the compound obtained in step b) of formula (I) in which $R_2$ = H and $R_3$ = $OCOCH_3$ is optionally treated in order to prepare a compound of formula (I) in which $R_2$ and $R_3$ together form a bond.

**6.** Pharmaceutical composition characterized in that it contains as active ingredient a compound of formula (I) according to any one of the claims 1 to 4.

**7.** Pharmaceutical composition according to claim 6, containing 0.01 to 2 mg of active ingredient per dosage unit.

**Claims for the following Contracting State : GR**

**1.** Compound of formula :

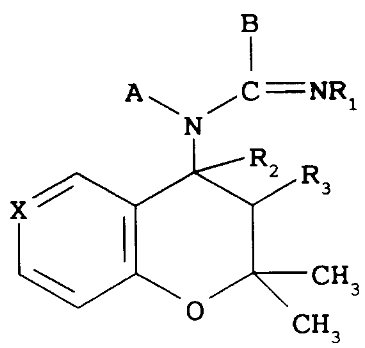

(I)

in which :

- A and B are linked together between N and C = $NR_1$ and represent
  - . either a -CH = CH-CH = N- group
  - . or a -CH = $CR_4$-$CR_5$ = CH- group, one of the substituents $R_4$ or $R_5$ denoting hydrogen, the other being selected from hydrogen, halogen, nitro or $C_1$-$C_4$ alkyl;
- X represents a nitrogen atom, a N-oxide group or the C-Z group;
- Z represents hydrogen, a halogen, a $C_1$-$C_4$ alkyl, a cyano group, a nitro group, an acetyl group or a trifluoroacetyl group, a phosphono or dialkoxyphosphoryl group, the alkyl group being a $C_1$-$C_3$ group or an amino group;
- $R_1$ represents hydrogen, a cyano group, a nitro group, a $C_1$-$C_4$ alkyl, an hydroxyl, a $C_1$-$C_4$ alkoxy, a trifluoroacetyl, a methanesulfonyl group, a benzensulfonyl group unsubstituted or substituted on the phenyl by a methyl, a halogen or a trifluoromethyl;
- $R_2$ represents hydrogen;
- $R_3$ represents an hydroxyl or acetyloxy group or $R_2$ and $R_3$ together form a bond.

**2.** Compound according to claim 1, of formula (I), in which :

- $R_1$ represents CN,

- X represents the C-$NO_2$ group or the C-CN group,
- A and B represents a -CH = $CR_4$-$CR_5$ = CH- group and
- $R_2$ and $R_3$ together form a bond.

**3.** Compound according to one of claims 1 or 2, of formula (I), characterized in that said compound is 4-(2-cyanimino 1,2-dihydro 1-pyridyl) 2,2-dimethyl 6-nitro chromene.

**4.** Compound according to one of claims 1 or 2, of formula (I), characterized in that said compound is 4-(2-cyanimino 1,2-dihydro 1-pyridyl) 6-cyano 2,2-dimethyl chromene.

**5.** Process for the preparation of a compound according to any one of claims 1 to 4, characterized in that :

a) an epoxide II of formula :

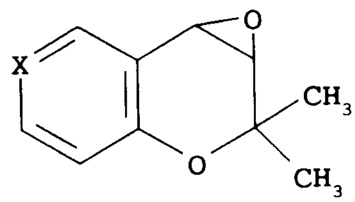

(II)

in which X has the meaning given in claim 1,is treated with a heterocycle III corresponding to one of the following tautomeric forms :

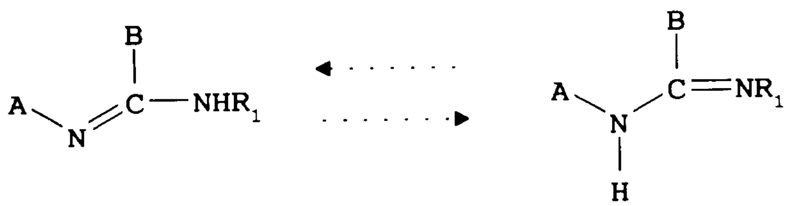

(III)

in which A, B and $R_1$ have the meanings indicated in claim 1,
b) acetic anhydride is optionally reacted with the compound obtained of formula (I) in which $R_2$ = H and $R_3$ = OH in order to prepare the compound of formula (I) in which $R_2$ = H and $R_3$ = $OCOCH_3$;
c) the compound obtained in step a) of formula (I) in which $R_2$ = H and $R_3$ = OH or the compound obtained in step b) of formula (I) in which $R_2$ = H and $R_3$ = $OCOCH_3$ is optionally treated in order to prepare a compound of formula (I) in which $R_2$ and $R_3$ together form a bond.

**6.** Process for the preparation of pharmaceutical compositions characterized in that it consists in including an efficient quantity of a compound of formula (I) according to any one of claims 1 to 4 in a suitable excipient.

**7.** Process according to claim 6 for the preparation of pharmaceutical compositions, containing 0.01 to 2 mg of active ingredient per dosage unit.

**Claims for the following Contracting State : ES**

**1.** Process for the preparation of compounds of formula :

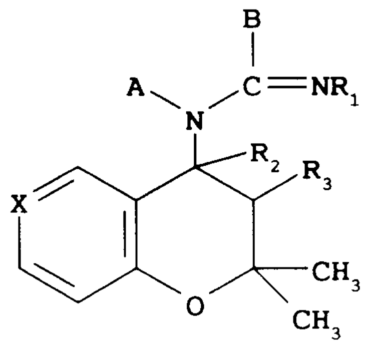

(I)

in which :

- A and B are linked together between N and $C=NR_1$ and represent
  - . either a -CH=CH-CH=N- group
  - . or a $-CH=CR_4-CR_5=CH-$ group, one of the substituents $R_4$ or $R_5$ denoting hydrogen, the other being selected from hydrogen, halogen, nitro or $C_1$-$C_4$ alkyl;
- X represents a nitrogen atom, a N-oxide group or the C-Z group;
- Z represents hydrogen, a halogen, a $C_1$-$C_4$ alkyl, a cyano group, a nitro group, an acetyl group or a trifluoroacetyl group, a phosphono or dialkoxyphosphoryl group, the alkyl group being a $C_1$-$C_3$ group or an amino group;
- $R_1$ represents hydrogen, a cyano group, a nitro group, a $C_1$-$C_4$ alkyl, an hydroxyl, a $C_1$-$C_4$ alkoxy, a trifluoroacetyl, a methanesulfonyl group, a benzensulfonyl group unsubstituted or substituted on the phenyl by a methyl, a halogen or a trifluoromethyl;
- $R_2$ represents hydrogen;
- $R_3$ represents an hydroxyl or acetyloxy group or $R_2$ and $R_3$ together form a bond,

characterized in that :

a) an epoxide II of formula :

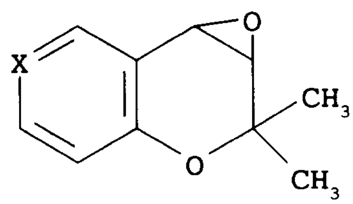

(II)

in which X has the meaning given above, is treated with a heterocycle III corresponding to one of the following tautomeric forms :

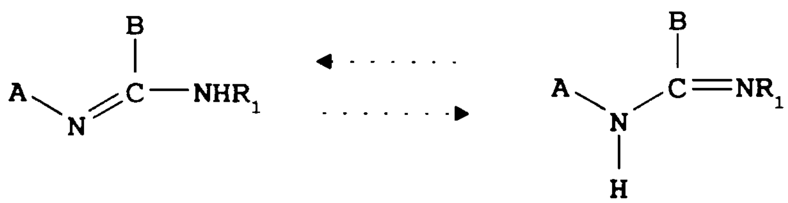

(III)

in which A, B and $R_1$ have the meanings indicated above,

b) acetic anhydride is optionally reacted with the compound obtained of formula (I) in which $R_2=H$ and $R_3=OH$ in order to prepare the compound of formula (I) in which $R_2=H$ and $R_3=OCOCH_3$;

c) the compound obtained in step a) of formula (I) in which $R_2 = H$ and $R_3 = OH$ or the compound obtained in step b) of formula (I) in which $R_2 = H$ and $R_3 = OCOCH_3$ is optionally treated in order to prepare a compound of formula (I) in which $R_2$ and $R_3$ together form a bond.

**2.** Process according to claim 1, characterized in that an epoxide of formula (II) in which X is the group C-$NO_2$ or the group C-CN is treated with an heterocycle of formula (III) in which A and B represent the group $-CH=CR_4-CR_5=CH-$ and $R_1$ is the group CN to prepare the compounds of formula (I) in which :
- $R_1$ represents CN,
- X represents the group C-$NO_2$ or the C-CN,
- A and B represents the group $-CH=CR_4-CR_5=CH-$and
- $R_2$ and $R_3$ together form a bond.

**3.** Process according to claim 1, characterized in that :
a) an epoxide of formula (II) in which X is the group C-$NO_2$ is treated with an heterocycle of formula (III) in which A and B represent the group -CH=CH-CH=CH- and $R_1$ is the group CN;
b) acetic anhydride is optionally reacted with the compound obtained of formula (I) in which $R_2 = H$ and $R_3 = OH$ in order to prepare the compound of formula (I) in which $R_2 = H$ and $R_3 = OCOCH_3$;
c) the compound obtained in step a) is treated with an alkaline hydride in inert solvent at a temperature comprised between 20 and 100 °C or optionally the compound obtained in step b) is treated by heating to between 50 and 110 °C in an inert solvent in the presence of diazabicycloundécène to obtain the 4-(2-cyanimino 1,2-dihydro 1-pyridyl) 2,2-dimethyl 6-nitro chromene.

**4.** Process according to claim 1, characterized in that
a) an epoxide of formula (II) in which X is the group C-CN is treated with an heterocycle of formula (III) in which A and B represent the group -CH=CH-CH=CH- and $R_1$ is the group CN;
b) acetic anhydride is optionally reacted with the compound obtained of formula (I) in which $R_2 = H$ and $R_3 = OH$ in order to prepare the compound of formula (I) in which $R_2 = H$ and $R_3 = OCOCH_3$;
c) the compound obtained in step a) is treated with an alkaline hydride in inert solvent at a temperature comprised between 20 and 100 °C or optionally the compound obtained in step b) is treated by heating to between 50 and 110 °C in an inert solvent in the presence of diazabicycloundécène to obtain the 4-(2-cyanimino 1,2-dihydro 1-pyridyl) 6-cyano 2,2-dimethyl chromene.

**5.** Process for the preparation of pharmaceutical compositions characterized in that it consists in including an efficient quantity of a compound of formula (I), prepared according to any one of claims 1 to 4, in a suitable excipient.

**6.** Process according to claim 5 for the preparation of pharmaceutical compositions containing 0.01 to 2 mg of active ingredient per dosage unit.

**Patentansprüche**
**Patentansprüche für folgende Vertragsstaaten : AT, BE, CH, DE, DK, FR, GB, IT, LI, LU, NL, SE**

**1.** Verbindung der Formel :

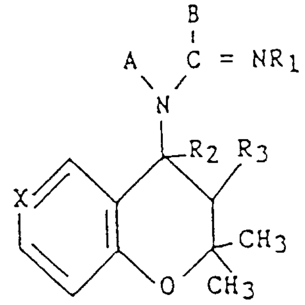

(I),

worin A und B Verbindungen sind zwischen N und $C=NR_1$ und entweder eine Gruppe -CH=CH-CH=N- oder eine Gruppe $-CH=CR_4-CR_5=CH-$ darstellen, wobei einer der Substituenten $R_4$ oder $R_5$

Wasserstoff bedeutet, und der andere ausgewählt ist aus Wasserstoff, Halogen, Nitro oder $C_1$-$C_4$-Alkyl; X ein Stickstoffatom, eine N-Oxid-Gruppe oder die Gruppe C-Z darstellt; Z Wasserstoff, Halogen, $C_1$-$C_4$-Alkyl, eine Cyano-Gruppe, eine Nitro-Gruppe, eine Acetyl-Gruppe oder eine Trifluoracetyl-Gruppe, eine Phosphono- oder Dialkoxyphosphoryl-Gruppe, wobei die Alkyl-Gruppe $C_1$-$C_3$ ist, oder eine Amino-Gruppe bedeutet; $R_1$ Wasserstoff, eine Cyano-Gruppe, eine Nitro-Gruppe, $C_1$-$C_4$-Alkyl, Hydroxyl, $C_1$-$C_4$-Alkoxy, Trifluoracetyl, eine Methansulfonyl-Gruppe, eine Benzolsulfonyl-Gruppe, unsubstituiert oder am Phenyl durch Methyl Halogen oder Trifluormethyl substituiert,darstellt; $R_2$ ein Wasserstoffatom ist; und $R_3$ eine Hydroxyl-Gruppe oder eine Acetyloxy-Gruppe bedeutet, oder $R_2$ und $R_3$ gemeinsam eine Bindung bilden.

**2.** Verbindung der Formel (I) nach Anspruch 1, worin $R_1$ die Bedeutung CN hat, X die Gruppe C-$NO_2$ oder die Gruppe C-CN darstellt, A und B eine Gruppe -CH=$CR_4$-$CR_5$=CH- sind, und $R_2$ und $R_3$ gemeinsam eine Bindung bilden.

**3.** Verbindung der Formel (I) nach Anspruch 1 oder 2, dadurch gekennzeichnet, daß sie 4-(2-Cyanimino-1,2-dihydro-1-pyridyl)-2,2-dimethyl-6-nitrochromen ist.

**4.** Verbindung der Formel (I) nach Anspruch 1 oder 2, dadurch gekennzeichnet, daß sie 4-(2-Cyanimino-1,2-dihydro-1-pyridyl)-6-cyano-2,2-dimethylchromen ist.

**5.** Verfahren zur Herstellung einer Verbindung nach einem der Ansprüche 1 bis 4, dadurch gekennzeichnet, daß:

a) ein Epoxid der Formel (II)

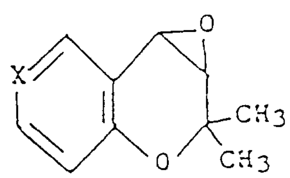

(II),

worin X die in Anspruch 1 angegebene Bedeutung hat, mit einem Heterocyclus (III) einer der folgenden tautomeren Formen

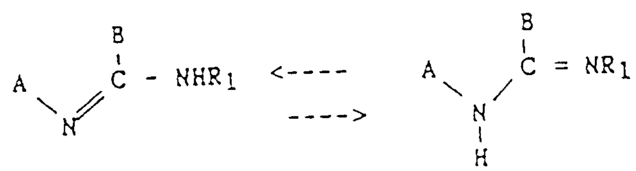

(III),

worin A, B und $R_1$ die in Anspruch 1 angegebenen Bedeutungen haben, behandelt wird;

b) gegebenenfalls die erhaltene Verbindung der Formel (I), worin $R_2$ = H und $R_3$ = OH, mit Essigsäureanhydrid umgesetzt wird, wobei die Verbindung der Formel (I), worin $R_2$ = H und $R_3$ = $OCOCH_3$, hergestellt wird;

c) gegebenenfalls die in Schritt a) erhaltene Verbindung der Formel (I), worin $R_2$ = H und $R_3$ = OH, oder die in Schritt b) erhaltene Verbindung der Formel (I), worin $R_2$ = H und $R_3$ = $OCOCH_3$, behandelt wird, wobei eine Verbindung der Formel (I), worin $R_2$ und $R_3$ gemeinsam eine Bindung bilden, hergestellt wird.

**6.** Pharmazeutische Zusammensetzung, dadurch gekennzeichnet, daß sie als aktiven Bestandteil eine Verbindung der Formel (I) nach einem der Ansprüche 1 bis 4 enthält.

**7.** Pharmazeutische Zusammensetzung nach Anspruch 6, welche 0,01 bis 2 mg aktiven Bestandteil pro Dosierungseinheit enthält.

**Patentansprüche für folgenden Vertragsstaat : GR**

1. Verbindung der Formel :

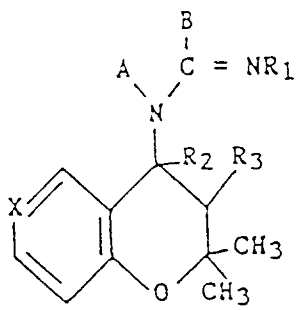

(I),

worin A und B Verbindungen sind zwischen N und $C=NR_1$ und entweder eine Gruppe $-CH=CH-CH=N-$ oder eine Gruppe $-CH=CR_4-CR_5=CH-$ darstellen, wobei einer der Substituenten $R_4$ oder $R_5$ Wasserstoff bedeutet, und der andere ausgewählt ist aus Wasserstoff, Halogen, Nitro oder $C_1$-$C_4$-Alkyl; X ein Stickstoffatom, eine N-Oxid-Gruppe oder die Gruppe C-Z darstellt; Z Wasserstoff, Halogen, $C_1$-$C_4$-Alkyl, eine Cyano-Gruppe, eine Nitro-Gruppe, eine Acetyl-Gruppe oder eine Trifluoracetyl-Gruppe, eine Phosphono- oder Dialkoxyphosphoryl-Gruppe, wobei die Alkyl-Gruppe $C_1$-$C_3$ ist, oder eine Amino-Gruppe bedeutet; $R_1$ Wasserstoff, eine Cyano-Gruppe, eine Nitro-Gruppe, $C_1$-$C_4$-Alkyl, Hydroxyl, $C_1$-$C_4$-Alkoxy, Trifluoracetyl, eine Methansulfonyl-Gruppe, eine Benzolsulfonyl-Gruppe, unsubstituiert oder am Phenyl durch Methyl Halogen oder Trifluormethyl substituiert, darstellt; $R_2$ ein Wasserstoffatom ist; und $R_3$ eine Hydroxyl-Gruppe oder eine Acetyloxy-Gruppe bedeutet, oder $R_2$ und $R_3$ gemeinsam eine Bindung bilden.

2. Verbindung der Formel (I) nach Anspruch 1, worin $R_1$ die Bedeutung CN hat, X die Gruppe $C-NO_2$ oder die Gruppe C-CN darstellt, A und B eine Gruppe $-CH=CR_4-CR_5=CH-$ sind, und $R_2$ und $R_3$ gemeinsam eine Bindung bilden.

3. Verbindung der Formel (I) nach Anspruch 1 oder 2, dadurch gekennzeichnet, daß sie 4-(2-Cyanimino-1,2-dihydro-1-pyridyl)-2,2-dimethyl-6-nitrochromen ist.

4. Verbindung der Formel (I) nach Anspruch 1 oder 2, dadurch gekennzeichnet, daß sie 4-(2-Cyanimino-1,2-dihydro-1-pyridyl)-6-cyano-2,2-dimethylchromen ist.

5. Verfahren zur Herstellung einer Verbindung nach einem der Ansprüche 1 bis 4, dadurch gekennzeichnet, daß:
   a) ein Epoxid der Formel (II)

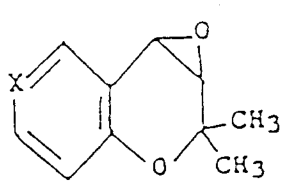

(II),

worin X die in Anspruch 1 angegebene Bedeutung hat, mit einem Heterocyclus (III) einer der folgenden tautomeren Formen

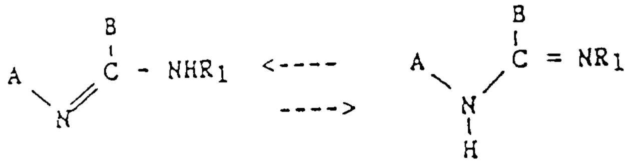

(III),

worin A, B und $R_1$ die in Anspruch 1 angegebenen Bedeutungen haben, behandelt wird;

b) gegebenenfalls die erhaltene Verbindung der Formel (I), worin $R_2$ = H und $R_3$ = OH, mit Essigsäureanhydrid umgesetzt wird, wobei die Verbindung der Formel (I), worin $R_2$ = H und $R_3$ = $OCOCH_3$, hergestellt wird;

c) gegebenenfalls die in Schritt a) erhaltene Verbindung der Formel (I), worin $R_2$ = H und $R_3$ = OH, oder die in Schritt b) erhaltene Verbindung der Formel (I), worin $R_2$ = H und $R_3$ = $OCOCH_3$, behandelt wird, wobei eine Verbindung der Formel (I), worin $R_2$ und $R_3$ gemeinsam eine Bindung bilden, hergestellt wird.

**6.** Verfahren zur Herstellung pharmazeutischer Zusammensetzungen, dadurch gekennzeichnet, daß es darin besteht, eine wirksame Menge einer Verbindung der Formel (I) nach einem der Ansprüche 1 bis 4 in einen geeigneten Exzipienten einzuschließen.

**7.** Verfahren nach Anspruch 6 zur Herstellung pharmazeutischer Zusammensetzungen, die 0,01 bis 2 mg aktiven Bestandteil pro Dosierungseinheit enthalten.

**Patentansprüche für folgenden Vertragsstaat : ES**

**1.** Verfahren zur Herstellung von Verbindungen der Formel:

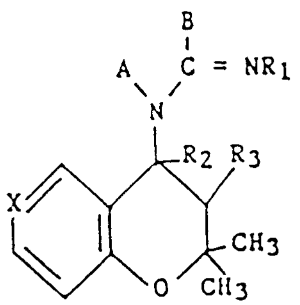

(I),

worin A und B Verbindungen sind zwischen N und C = $NR_1$ und entweder eine Gruppe -CH = CH-CH = N- oder eine Gruppe -CH = $CR_4$-$CR_5$ = CH- darstellen, wobei einer der Substituenten $R_4$ oder $R_5$ Wasserstoff bedeutet, und der andere ausgewählt ist aus Wasserstoff, Halogen, Nitro oder $C_1$-$C_4$-Alkyl; X ein Stickstoffatom, eine N-Oxid-Gruppe oder die Gruppe C-Z darstellt; Z Wasserstoff, Halogen, $C_1$-$C_4$-Alkyl, eine Cyano-Gruppe, eine Nitro-Gruppe, eine Acetyl-Gruppe oder eine Trifluoracetyl-Gruppe, eine Phosphono- oder Dialkoxyphosphoryl-Gruppe, wobei die Alkyl-Gruppe $C_1$-$C_3$ ist, oder eine Amino-Gruppe bedeutet; $R_1$ Wasserstoff, eine Cyano-Gruppe, eine Nitro-Gruppe, $C_1$-$C_4$-Alkyl, Hydroxyl, $C_1$-$C_4$-Alkoxy, Trifluoracetyl, eine Methansulfonyl-Gruppe, eine Benzolsulfonyl-Gruppe, unsubstituiert oder am Phenyl durch Methyl , Halogen oder Trifluormethyl substituiert , darstellt; $R_2$ ein Wasserstoffatom ist; und $R_3$ eine Hydroxyl-Gruppe oder eine Acetyloxy-Gruppe bedeutet, oder $R_2$ und $R_3$ gemeinsam eine Bindung bilden,

dadurch gekennzeichnet, daß

a) ein Epoxid der Formel (II)

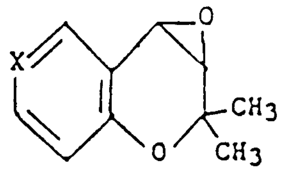

(II),

worin X die oben angegebene Bedeutung hat, mit einem Heterocyclus (III) einer der folgenden tautomeren Formen

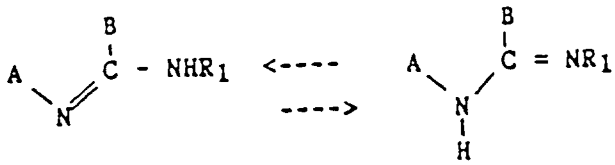

(III),

worin A, B und $R_1$ die oben angegebenen Bedeutungen haben, behandelt wird;
b) gegebenenfalls die erhaltene Verbindung der Formel (I), worin $R_2$ = H und $R_3$ = OH, mit Essigsäureanhydrid umgesetzt wird, wobei die Verbindung der Formel (I), worin $R_2$ = H und $R_3$ = $OCOCH_3$, hergestellt wird;
c) gegebenenfalls die in Schritt a) erhaltene Verbindung der Formel (I), worin $R_2$ = H und $R_3$ = OH, oder die in Schritt b) erhaltene Verbindung der Formel (I), worin $R_2$ = H und $R_3$ = $OCOCH_3$, behandelt wird, wobei eine Verbindung der Formel (I), worin $R_2$ und $R_3$ gemeinsam eine Bindung bilden, hergestellt wird.

**2.** Verfahren nach Anspruch 1, dadurch gekennzeichnet, daß ein Epoxid der Formel (II), worin X die Gruppe $C\text{-}NO_2$ oder die Gruppe C-CN darstellt, mit einem Heterocyclus der Formel (III), worin A und B eine Gruppe $-CH=CR_4-CR_5=CH-$ sind, und $R_1$ die Gruppe CN bedeutet, behandelt wird, wobei die Verbindungen der Formel (I), worin $R_1$ die Bedeutung CN hat, X die Gruppe $C\text{-}NO_2$ oder die Gruppe C-CN darstellt, A und B eine Gruppe $-CH=CR_4-CR_5=CH-$ sind, und $R_2$ und $R_3$ gemeinsam eine Bindung bilden, hergestellt werden.

**3.** Verfahren nach Anspruch 1, dadurch gekennzeichnet, daß:
a) ein Epoxid der Formel (II), worin X die Gruppe $C\text{-}NO_2$ darstellt, mit einem Heterocyclus der Formel (III), worin A und B die Gruppe -CH=CH-CH=CH- sind, und $R_1$ die Gruppe CN bedeutet, behandelt wird;
b) gegebenenfalls die erhaltene Verbindung der Formel (I), worin $R_2$ = H und $R_3$ = OH, mit Essigsäureanhydrid reagieren gelassen wird, wobei die Verbindung der Formel (I), worin $R_2$ = H und $R_3$ = $OCOCH_3$, hergestellt wird;
c) die in Schritt a) erhaltene Verbindung mit einem Alkalihydrid in einem inerten Lösungsmittel bei einer Temperatur zwischen 20 und 100 °C oder gegebenenfalls die in Schritt b) erhaltene Verbindung durch Erhitzen auf 50 bis 110 °C in einem inerten Lösungsmittel in Anwesenheit von Diazabicycloundecen behandelt wird, wobei 4-(2-Cyaniminol-1,2-dihydro-1-pyridyl)-2,2-dimethyl-6-nitrochromen erhalten wird.

**4.** Verfahren nach Anspruch 1, dadurch gekennzeichnet, daß:
a) ein Epoxid der Formel (II), worin X die Gruppe C-CN darstellt, mit einem Heterocyclus der Formel (III), worin A und B die Gruppe -CH=CH-CH=CH- sind, und $R_1$ die Gruppe CN bedeutet, behandelt wird;
b) gegebenenfalls die erhaltene Verbindung der Formel (I), worin $R_2$ = H und $R_3$ = OH, mit Essigsäureanhydrid reagieren gelassen wird, wobei die Verbindung der Formel (I), worin $R_2$ = H und $R_3$ = $OCOCH_3$, hergestellt wird;

c) die in Schritt a) erhaltene Verbindung mit einem Alkalihydrid in einem inerten Lösungsmittel bei einer Temperatur zwischen 20 und 100 ° C oder gegebenenfalls die in Schritt b) erhaltene Verbindung durch Erhitzen auf 50 bis 110 ° C in einem inerten Lösungsmittel in Anwesenheit von Diazabicycloundecen behandelt wird, wobei 4-(2-Cyanimino-1,2-dihydro-1-pyridyl)-6-cyano-2,2-dimethylchromen erhalten wird.

**5.** Verfahren zur Herstellung pharmazeutischer Zusammensetzungen, dadurch gekennzeichnet, daß es darin besteht, eine wirksame Menge einer Verbindung der Formel (I), hergestellt nach einem der Ansprüche 1 bis 4, in einen geeigneten Exzipienten einzuschließen.

**6.** Verfahren nach Anspruch 5 zur Herstellung pharmazeutischer Zusammensetzungen, die 0,01 bis 2 mg aktiven Bestandteil pro Dosierungseinheit enthalten.